(19) Europäisches Patentamt
European Patent Office
Office européen des brevets

(11) Publication number: **0 347 964 A1**

(12) **EUROPEAN PATENT APPLICATION**

(21) Application number: 89201441.6

(22) Date of filing: 06.06.89

(51) Int. Cl.4: C07H 15/04 , A61K 31/70

(30) Priority: 21.06.88 EP 88201270

(43) Date of publication of application: 27.12.89 Bulletin 89/52

(84) Designated Contracting States: AT BE CH DE ES FR GB GR IT LI LU NL SE

(71) Applicant: AKZO N.V.
Velperweg 76
NL-6824 BM Arnhem(NL)

Applicant: SANOFI S.A.
40, Avenue George V
F-75008 Paris(FR)

(72) Inventor: Van Boeckel, Constant Adriaan Anton
Mercuriusstraat 32
NL-5346 LX Oss(NL)

(74) Representative: Hermans, Franciscus G.M.
Patent Department AKZO N.V. Pharma Division P.O. Box 20
NL-5340 BH Oss(NL)

(54) **New oligosaccharides with internal spacer.**

(57) The present invention is dealing with new saccharides of the formula I:

I

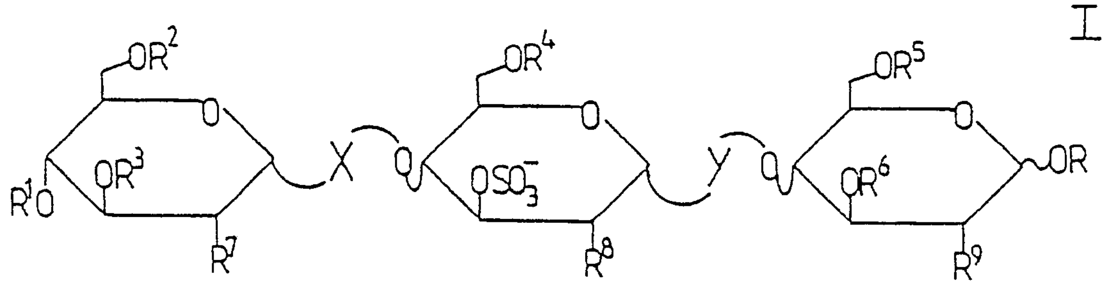

1 2 3 4 5

wherein
X and Y represent a moiety selected from an optionally sulphated uronic acid moiety and a non-uronic acid spacer which consists of a linear or cyclic chain of atoms and carries at least one electronegatively charged substituent preferably a carboxylate group, provided that at least one of the moieties X or Y represents the said non-uronic acid spacer,
R is hydrogen or alkyl (1-20 C),
$R^1$, $R^2$, $R^3$, $R^4$, $R^5$ and $R^6$ represent hydrogen or the moiety $SO_3^-$,
$R^7$, $R^8$, $R^9$ represent OH, $OSO_3^-$, $NHSO_3^-$ or NH-acyl,
These saccharides have anti-thrombotic activity and especially they activate thrombin via AT-III.
The invention also refers to new intermediates in the synthesis of the above saccharides.

EP 0 347 964 A1

# New oligosaccharides with internal spacer

The present invention is dealing with new saccharides, with a process for their preparation and with pharmaceutical compositions containing same.

The invention is also dealing with new intermediates in the preparation of the new saccharides.

More particularly the present invention is dealing with saccharides of the formula I:

I

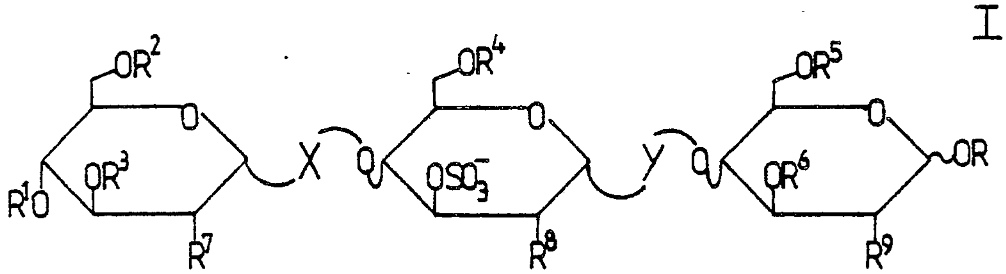

1 2 3 4 5

wherein

X and Y represent a moiety selected from an optionally sulphated uronic acid moiety and a non-uronic acid spacer which consists of a linear or cyclic chain of atoms and carries at least one electronegatively charged substituent preferably a carboxylate group, provided that at least one of the moieties X or Y represents the said non-uronic acid spacer,

R is hydrogen or alkyl (1-20 C),

$R^1$, $R^2$, $R^3$, $R^4$, $R^5$ and $R^6$ represent hydrogen or the moiety $SO_3^-$,

$R^7$, $R^8$, $R^9$ represent OH, $OSO_3^-$, $NHSO_3^-$ or NH-acyl, and the charge of the various charged moieties is compensated by a suitable (pharmaceutically acceptable) counter-ion, which may be hydrogen but is preferably an alkali or earth-alkali metal ion.

These new synthetic oligosaccharides have a short biological half-life while retaining an antithrombotic activity, which is of advantage for certain applications, particularly in extra-corporeal circulation, such as renal dialysis and hemodialysis and as anticoagulant in cardiopulmonary bypass. These oligosaccharides may also be used as laboratory reagents.

The unique anti-thrombin III (AT-III) binding region of the anti-coagulant drug heparin consists of a sulphated pentasaccharide which catalyzes the AT-III mediated inactivation of the coagulation factor Xa. It has been established that most of the charged groups present in the above-meant pentasaccharide participate in the AT-III activation process, so that it was generally thought that the complex carbohydrate hexapyranose building blocks - as present in the pentasaccharide -were necessary to retain potent anti-Xa activity. In this context reference is made to European (published) patent application 84.999 and to copending European patent application 87.201.383.4 where highly active synthetic pentasaccharides are described.

Surprisingly it was found now that the building blocks 2 and/or 4 of the original pentasaccharide (indicated by the building block sequence 1-2-3-4-5) do not necessarily need to be the optionally sulphated uronic acid building blocks, viz. and β-D-glucuronic acid moiety of building block 2 and the α-L-iduronic acid moiety of building block 4, as present in the original pentasaccharide. The said uronic acid building blocks may be replaced by a non-uronic acid linking group or spacer which consists of a linear or cyclic chain of atoms preferably composed of carbon, oxygen and hydrogen and which chain carries at least an electonegatively charged substituent, preferably a carboxylate moiety. The said spacer is bridging the distance between the anomeric oxygen atom of building block 1 and the oxygen atom at position 4 of building block 3 and/or the distance between the anomeric oxygen atom of building block 3 and the oxygen atom 4 of building block 5, said distance being in between about 3 and about 8 Angstrom units (in a molecular model).

An excellent spacer - as meant in the definition of X and/or Y, is a group of the formula II:

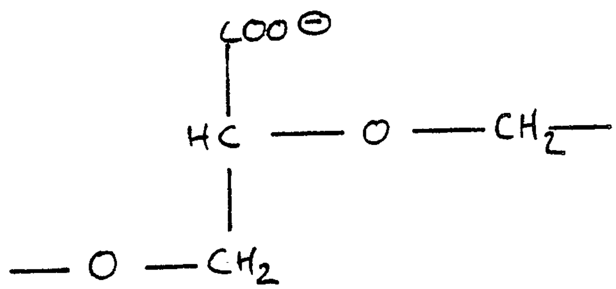

II

This spacer of formula II has a chiral center, so that the R- or the S-configuration can be applied, whereby the R-configuration is the preferred configuration to replace building block 4 and the S-configuration to replace building block 2.

According to the present invention at least one of the building blocks 2 and 4 of the original pentasaccharide must be replaced by the above identified spacer X and/or Y. That building block (2 or 4), that is not replaced by the said spacer is an uronic acid moiety, whereby the β-D-glucuronic acid moiety is the preferred uronic acid moiety for building block 2 and the α-L-iduronic acid moiety the preferreed uronic acid moiety for building block 4.

The most preferred compound according to the general formula I is a compound, in which X represents an uronic acid moiety and more preferably the β-D-glucuronic acid moiety, and Y represents the spacer of formula II and more preferably the R-configuration of spacer II.

The saccharides according to the invention are prepared according to well known methods described and used for the synthesis of polysaccharides. See for example the European patent applications referred to already.

In general the final saccharides according to formula I are prepared starting from a new intermediate compound of the formula III:

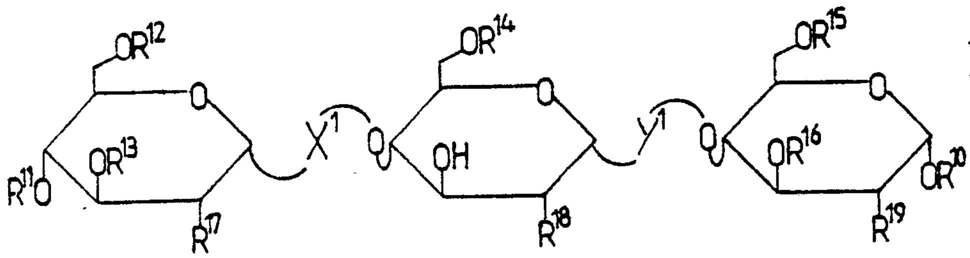

III

in which
$R^{10}$ represents a hydroxyl protecting group or alkyl (1-20C)
$R^{11}$, $R^{12}$, $R^{13}$, $R^{14}$, $R^{15}$, and $R^{16}$ represent hydrogen or a hydroxyl protecting group, preferably a group that can be removed by hydrogenolysis, such as a benzyl group,
$R^{17}$, $R^{18}$ and $R^{19}$ represent a hydroxyl group, a protected hydroxyl group, an azido group ($N_3$), and NH-acyl moiety or an amino moiety that is protected by a protecting group which can be removed by hydrogenolysis,
$X'$ and $Y'$ represent an optionally protected uronic acid moiety or the spacer defined in the definition of X and Y, reactive group(s) of said spacer being optionally protected, provided that one of the moieties $X'$ or $Y'$ represents said spacer moiety, or a salt thereof.

The intermediate product according to formula III can be converted into the final saccharide of formula I by methods well known in carbohydrate chemistry and described for various analogous saccharides, e.g. by the following subsequent steps:

1) sulphation of the free hydroxyl groups,

2) removal, preferably by hydrogenolysis, of the hydroxyl-protecting groups resulting in free hydroxyl groups and simultaneous hydrogenolysis of the N-protecting groups, such as the azido or N-benzyloxycarbonyl group(s), if present in the molecule, to the $-NH_2$ moiety,

3) sulphation of the $-NH_2$ moiety(ies) thus obtained.

In an alternative but in essence similar method, the above sequence of steps can be interchanged, viz.

1) reduction of the azido moiety(ies) and other reducable groups - if present - resulting in (a) free amino group(s),

2) simultaneous sulphation of both the available free hydroxyl groups and free $NH_2$ groups and

3) hydrogenolysis or otherwise removal of the hydroxyl protecting groups.

The sulphation of a hydroxyl group is well known in the art and can be carried out for example by dissolving the product in a suitable (inert) solvent such as dimethylformamide and adding a suitable sulphating agent such as sulphur trioxide, and preferably a tri(m)ethylamine-sulphur trioxide complex.

The sulphation of an amino group ($NH_2$ group) may be carried out in the same manner, whereby an alkaline pH of the reaction mixture and preferably above pH 8 is required.

The hydrogenolysis of the hydroxyl-protecting group is carried out in a manner well known in the art and described in the chemical text books. A preferred hydrogenolysis is carried out by agitating the reaction mixture with hydrogen in the present of a metal catalyst such as platina, palladium or palladium on charcoal.

The preferred hydroxyl protecting group that can be removed by hydrogenolysis is benzyl.

The azido group may be hydrogenolyzed in the same manner but may - where a simultaneous hydrogenolysis together with the hydroxyl-protecting group is not necessary or wanted - also be selectively converted into the $-NH_2$-moiety by other reduction means such as metal hydrids e.g. lithium aluminium hydride or sodium borohydride, or with $H_2S$ in pyridine.

The compounds of the invention are preferably isolated in the form of a pharmaceutically acceptable salt, especially an alkali metal or earth-alkali metal salt, such as sodium, potassium, lithium or calcium.

The new intermediate saccharide of formula III may be prepared in a similar manner as described already in the literature for analogous saccharides by condensing the necessary building blocks 1,2,3,4, and 5 in the correct order whereby in contrast to the prior art either building block 2 or building block 4 or both building blocks 2 and 4 are no longer a building block derived from an uronic acid, but a building block based on the afore-identified non-uronic acid spacer.

As already said before, the preferred spacer is a group of the formula II. This preferred spacer may be derived from a suitable protected and activated (R) or (S) - glyceric acid derivative indicated by the formula IV

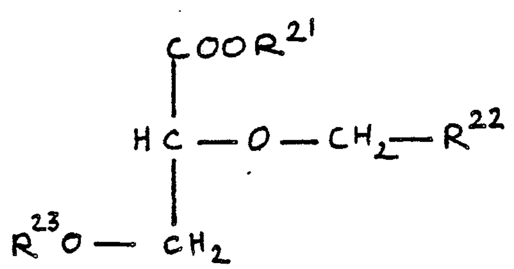

IV

wherein

$R^{21}$ is a carboxyl protecting group, preferably a lower alkyl group, e.g. methyl,

$R^{22}$ is a reactive leaving group, preferably imidate or halogen, e.g. fluorine, chlorine, bromine or trichloroacetimidate; and

$R^{23}$ is a hydroxyl protecting group, preferably of the type that can be removed through hydrolysis, e.g. allyl.

In a preferred process for the preparation of the intermediate saccharide of formula III, a suitable protected saccharide derivative consisting of the building block sequence 2-3 is activated at the anomeric center of building block 3 and is then coupled to a suitable protected saccharide derivative consisting of building block sequence 4-5, resulting in a protected saccharide derivative consisting of the building block sequence 2-3-4-5.

This saccharide derivative is subsequently deprotected selectively at the 4th position of the uronic acid building block 2 or - if building block 2 represents the spacer group - the protecting group $R^{23}$ (formula IV) is removed, after which the resulting saccharide derivative is then coupled with an activated and suitably protected building block 1 resulting in a protected saccharide derivative consisting of building block sequence 1-2-3-4-5. Subsequent hydrolysis or otherwise deprotection of at least the protected hydroxyl group at 3rd position of building block 3 result in an intermediate saccharide of formula III.

The said saccharide derivative of the building block sequence 2-3 and the saccharide derivative 4-5 are new intermediates, in as far as building block 2 and building block 4 are derived from the said non-uronic acid spacer.

The new intermediate 2-3 and the new intermediate 4-5 and salts thereof are characterised by the general formula V

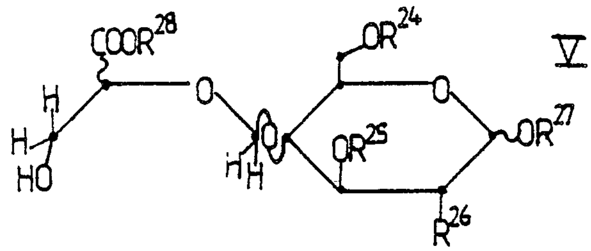

V

wherein

$R^{24}$ and $R^{25}$ represent hydrogen or a hydroxyl protecting group,

$R^{26}$ represents a hydroxyl group, a protected hydroxyl group, an azido group, an $NH_2$ group or or a N-protected-amino group,

$R^{27}$ represents hydrogen, an hydroxyl protecting group or alkyl (1-20 C); and

$R^{28}$ represents hydrogen or a carboxyl-protecting group.

This new intermediate of formula V may be prepared by reaction of the glyceric acid derivative of formula IV with a suitably protected monosaccharide of the formula VI

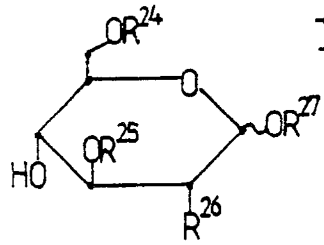

VI

wherein $R^{24}$, $R^{25}$, $R^{26}$ and $R^{27}$ have the aforesaid meanings after which the compound thus obtained is at least partially deprotected to remove the protecting group $R^{23}$.

Other new intermediates in the synthesis of the saccharide according to formula III are saccharide derivatives consisting of the building block sequence 2-3-4-5. The latter new intermediates and salts thereof are characterised by the general formulae: VII A, VII B and VII C

VII A

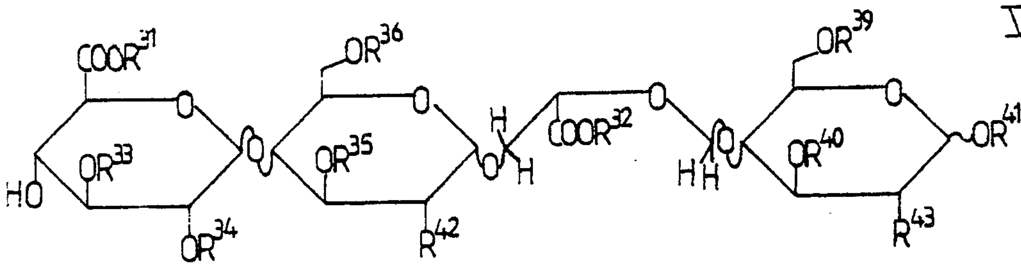

2 3 4 5

VII B

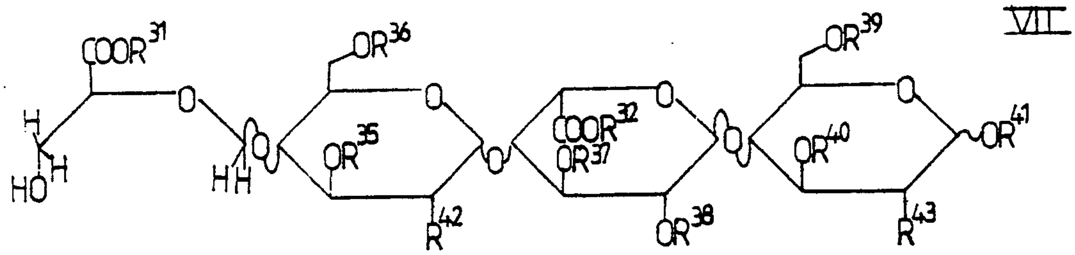

2 3 4 5

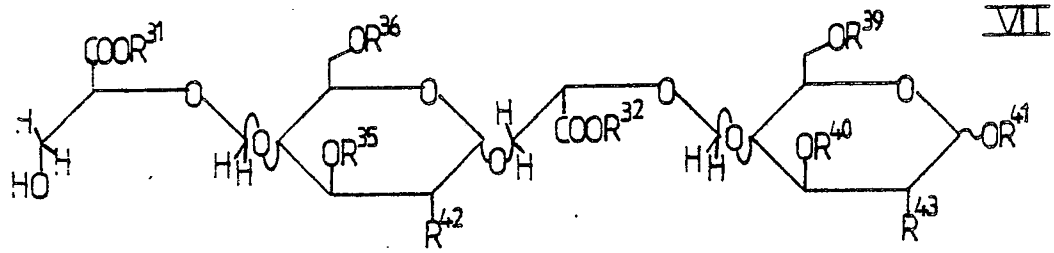

VII C

2 3 4 5

wherein

$R^{31}$ and $R^{32}$ represent hydrogen or a carboxyl protecting group,

$R^{33}$, $R^{34}$, $R^{35}$, $R^{36}$, $R^{37}$, $R^{38}$, $R^{39}$ and $R^{40}$ represent hydrogen or a hydroxyl protecting group,

$R^{41}$ represents hydrogen, a hydroxyl protecting group or an alkyl (1-20 C) group,

$R^{42}$ and $R^{43}$ represent hydroxy, a protected hydroxyl group, an azido group, an amino group or a N-protected amino group.

The new intermediate of formula VII A may be obtained by coupling the (activated) known dissacharide unit 2-3 to a saccharide derivative of formula V, after which at least the terminal hydroxyl group (4th position of building block 2) is deprotected.

In an almost analogous manner the intermediates of formula VII B and VII C can be prepared.

In an alternative synthesis for the preparation of the intermediate saccharide of formula III, a suitably protected building block 3 having an activated anomeric center is reacted with a glyceric acid derivative resulting in a compound of the formula VIII or a salt thereof,

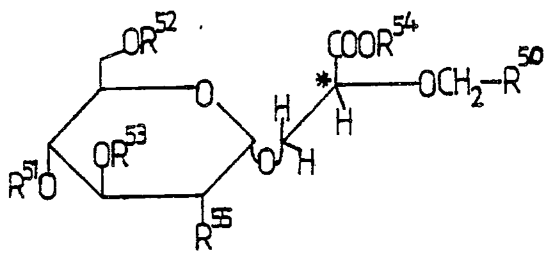

VIII

wherein

$R^{51}$, $R^{52}$ and $R^{53}$ represent hydrogen or a hydroxyl protecting group,

$R^{54}$ represents hydrogen or a carboxyl protecting group,

$R^{55}$ represents a hydroxyl group, a protected hydroxyl group, an azido group, an amino group or a N-protected amino group; and

$R^{50}$ represents a leaving group, e.g. imidate or halogen and preferably fluorine or trichloroacetimidate.

The product of formula VIII can be obtained as a mixture of the diastereoisomers R and S and can be separated in the individual diastereoisomers (* is R or S). The R-form of formula VIII is subsequently reacted with a suitably protected building block 5, resulting in a product having the sequence 3-4-5, after which at least the hydroxyl group at position 4 of building block 3 is removed resulting in an intermediate product of the general formula IX or a salt thereof

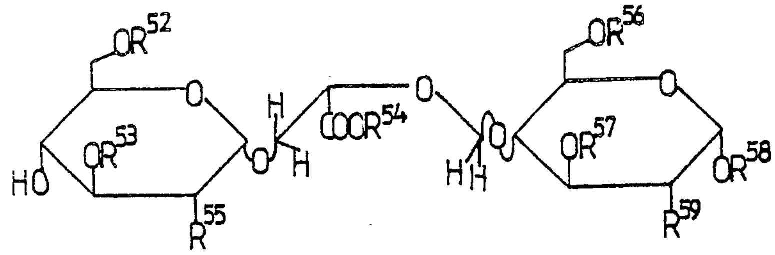

IX

wherein

$R^{52}$, $R^{53}$, $R^{54}$ and $R^{55}$ have the meanings indicated before, $R^{56}$ and $r^{57}$ represent hydrogen or a hydroxyl protecting group, $R^{58}$ represents hydrogen, a hydroxyl protecting group or an alkyl group (1-20 C) and $R^{59}$ has the same meansing as given already for $R^{55}$.

This intermediate product of formula IX is then coupled with the product of formula VIII (now preferably in the S-form) resulting in a protected product with the sequence 1-2-3-4-5. Subsequent hydrolysis or otherwise deprotection of at least the protected hydroxyl group at 3rd position of building block 3 results in the intermediate saccharide of formula III inwhich X′ and Y′ both are non-uronic acid spacers.

Coupling the intermediate IX with the disaccharide building block 1-2 finally results in an intermediate of formula III, in which only Y′ represents the non-uronic acid spacer.

Compounds according to formula I can also be prepared by different coupling strategies. For instance, compound 13a/b can be coupled with a derivative of compound 11, in which the bromide is replaced by e.g. an acetyl group and the levulinoyl group is replaced by hydrogen. The building block sequence 1-2-3 thus obtained is activated at the anomeric centre in the usual manner by a leaving group like e.g. bromine, fluorine or trichloroacetamide, and coupled with 10b, after which compound 14a/b is obtained, which can be converted to a compound of formula I using the hereinbefore described procedures. Other coupling combinations, e.g. 1-2 with 3-4-5 or stepwise 4-5→3-4-5→2-3-4-5→1-2-3-4-5, are also possible.

The preferred saccharide according to formula I possesses whether or not in combination:

a) a non-uronic acid spacer moiety for Y, preferably the spacer of formula II,

b) an alkyl group (1-4 C) and preferably methyl for R,

c) a sulphate moiety at least for $R^2$ and $R^5$,

d) an aminosulphate moiety or O-sulphate moiety for $R^7$, $R^8$ and $R^9$, and the saccharide is isolated in the form of its alkali metal salt, preferably the sodium salt.

The novel compounds of the invention can be employed both enterally (e.g. orally or rectally) and parenterally. For this purpose they are usually mixed with suitable pharmaceutical auxiliaries and then compounded into tablets, pills, dragees, pastilles, powders, capsules, microcapsules, suppositories, sprays (for example for intranasal administration), emulsions, suspensions or solutions.

These pharmaceutical preparations are manufactured in accordance with generally known galenical methods.

Parental administration is usually carried out with the aid of a syringe by means of which an emulsion, suspension or preferably a solution containing the active compound is administered subcutaneously, intramuscularly or intravenously.

The usual daily dosage, which can vary depending on the active compound used, is preferably between 0.01 -20 mg/kg bodyweight for enteral administration and 0.01 - 10 mg/kg for parenteral administration.

For a more detailed description of the various forms of administration reference is made to the European patent application 84,999.

In the following examples reference is made to the flow sheets A, B and C. The intermediates and end-products are not indicated by the chemical name but by reference to the corresponding compound in the flow sheets.

## Example 1

(Reference to flow sheets A and B)

Compound 2.

A solution of compound 1 [C.M. Lok, J.P. Ward and D.A. van Dorp, Chem. Phys. of Lipids 16, 115 (1976)] (4.05 g, 2813 mmol) in 70% acetic acid (70 ml) was stirred overnight at room temperature. The reaction mixture was evaporated to dryness and coevaporated with dry toluene. The residue was eluted from a silica column (200 g, dichloromethane/acetone, 98/2 → 8/2) to afford compound 2 (2.55 g, 84%). Rf = 0.45 (dichloromethane/acetone, 9/1 v/v).

Compound 3.

A solution of dimethyoxytritylchloride (DMT-chloride) (3.88 g, 11.46 mmol) in dry THF (51 ml) was added dropwise to a mixture of compound 2 (1.25 g, 10.42 mmol) in dry pyridine (51 ml) at a temperature of -15 °C.

After 16 hours the mixture was diluted with dichloromethane. Washed with water, saturated aqueous $NaHCO_3$ and brine and dried over a Whatmann phase separation filter. The filtrate was concentrated to dryness and coevaporated with dry toluene. The crude product was purified by silica chromatography (60 g, toluene/acetone, 95/5 → 8/2) to give compound 3 (2.07 g, 47%). Rf = 0.67 (toluene/acetone, 9/1).

Compound 4

Compound 3 (2.07 g, 4.9 mmol) was dissolved in dichloromethane (20 ml) and diisopropylethylamine (8.5 ml, 4.9 mmol) and stirred for 5 minutes at ambient temperature. Next, methyloxymethylchloride (MOM-chloride) (1.58 ml, 19.6 mmol) was added dropwise to the mixture whereafter the mixture was refluxed at 40 °C during 4 hours.

The reaction mixture was diluted with dichloromethane and washed with aqueous $NaHCO_3$, water and brine. The organic layer was filtered over a Whatmann phase separation filter and evaporated to dryness. The crude product was eluted from a silica column (60 g, toluene/acetone, 95/5) to give compound 4 (1.98 g, 87%). Rf = 0.44 (toluene/acetone, 95/5).

Compound 5

Compound 4 (3,5 g, 7.5 mmol) was dissolved in 80% acetic acid (50 ml) and stirred at room temperature. After 2 hours the mixture was diluted with water and pyridine and evaporated to a small volume.

Next, the mixture was diluted once more with pyridine and evaporated to dryness. Elution of the crude product from a silica column (50 g, dichloromethane/acetone, 99/1 → 9/1) afforded compound 5 (1.1 g, 89%) Rf = 0.12 (toluene/acetone, 9/1).

Compound 6

A solution of allyloxocarbonylchloride (320 µl, 3.0 mmol) in acetonitril (5 ml) was added dropwise to a mixture of compound 5 (415 mg, 2.5 mmol) in dry pyridine (20 ml) in approximately 30 minutes at a temperature of -35 °C.

After 2 hours the mixture was diluted with dichloromethane, washed with aqueous $NaHCO_3$, water and brine. The organic layer was dried ($MgSO_4$) and evaporated to dryness. After silica chromatography (10 g, dichloromethane/acetone, 99/1 → 98/2) compound 6 was isolated (450 mg, 73%). Rf = 0.50 (dichloromethane/acetone, 98/2).

Compound 7.

Compound 6 (450 mg, 1.8 mmol) was dissolved in dry dioxane (8 ml), under argon, after which a catalytic amount of palladium tetrakistriphenylphosphine was added. Then the mixture was refluxed during 20 minutes at a temperature of 110 °C. Thereafter the mixture was cooled to room temperature and evaporated to dryness. Purification over silica (7.5 g, dichloromethane/acetone, 99/1 → 98/2) gave compound 7 (330 mg, 82%). Rf = 0.32 (dichloromethane/acetone, 98/2, v/v).

Compound 8.

Compound 7 (1 g, 4.90 mmol) was dissolved in a mixture of acetic anhydride, acetic acid and trifluoro acetic acid (24 ml, 80/12/8) and stirred for 1 hour at room temperature.

Thereafter the mixture was concentrated and eluted from a silica column (25 g, toluene/acetone, 99/1 → 95/5). The product was coevaporated with dry toluene and dissolved in dichloromethane (15 ml). At a temperature of 0 °C, 70% HF in pyridine (3.5 ml) was added dropwise to this solution.

After 1 hour at 0 °C the reaction mixture was poured out in aqueous sodium acetate and dichloromethane and stirred for 5 minutes. The organic layer was washed with aqueous $NaHCO_3$ and brine and dried ($MgSO_4$). The crude product was purified over silica (20 g, toluene /acetone, 99/1 → 95/5) and afforded compound 8.

Compound 10a.

A mixture of compound 8 (340 mg, 1.75 mmol) and 9 (820 mg, 1.73 mmol) in dichloromethane (28 ml) was stirred for 1 hour at room temperature in the presence of activated mole sieves 4 Å. At a temperature of -20 °C a solution of 1 M $BF_3$-etherate in dichloromethane (1.75 ml) was added dropwise to the reaction mixture in approximately 5 minutes.

After 1 hour the reaction mixture was diluted with dichloromethane, filtered and washed with aqueous $NaHCO_3$ and brine.

The organic layer was dried ($MgSO_4$) and evaporated to dryness. The residue was purified by chromatography on silica (3,5 g, toluene/acetone, 99/1 → 9/1) and afforded the desired saccharide 10a (910 mg, 84%). Rf = 0.73 (toluene/acetone, 8/2, v/v), $[\alpha]_D^{20} = +107.9^\circ$ (c = 1.06, $CH_2Cl_2$).

Compound 10b. Intermediate compound according to formula V.

Compound 10a (400 mg, 0.62 mmol) was dissolved in acetic acid (1.4 ml) and water (0.07 ml). Under nitrogen sodium acetate (132 mg, 1.61 mmol) and palladium chloride (133 mg, 0.74 mmol) were added and the mixture was stirred overnight.

Then the mixture was diluted with dichloromethane, filtered, washed with water, aqueous $NaHCO_3$ and brine and subsequently dried ($MgSO_4$). The crude product was purified over $SiO_2$ (15 g, dichloromethane/acetone, 98/2 → 9/1) to give compound 10b (112 mg, 39%). Rf = 0.29 (dichloromethane/acetone, 9/1).

$^1$H-NMR ($CDCl_3$): 1.92 (s, 3H, $-OCH_3$), 2.82 (t, 1H, OH),

$$3.42\ (s,\ 3H,\ \overset{O}{C}-CH_3),\ 3.71\ (s,\ 3H,\ -\overset{O}{C}-OCH_3),$$

4.28 (dd, J = 3.8 and 6.0 Hz, 1H, -CH), 4.62 (c, 2H, H6a, H6b), 4.75 (d, J 4 Hz, 1H, H-1), 4.84 (AB, J 7 Hz, 2H, $-O-CH_2-O$), 5.09 (AB, J 12 Hz, 2 H, $-O-CH_2-C_5H_6$), 5.05 (d, J 10 Hz, 1H, -NH), 5.23 (dd, J 9 Hz and 10 Hz, 1 H, H-3).

"Trisaccharide" 12a.

To a solution of compound 10b (400 mg, 0.66 mmol) in dichloromethane (16 ml) were added molecular sieves (4Å, 1.2 g) under nitrogen. After stirring the mixture for 2 h at room temperature mercury (II) bromide (190 mg, 0.53 mmol) and mercury (II) cyanide (134 mg, 0.53 mmol) were added. The reaction mixture was cooled to -20 °C and a solution of bromide 11 [C.A.A. van Boeckel et al, J. Carb. Chem., 4, 293(1985)] (650 mg, 0.80 mmol) in dichloromethane (10 ml) was added dropwise over 30 min. After 48 h at room temperature, the mixture was filtered over Hyflo. The organic solution was washed with aqueous KBr (2.0 M), $NaHCO_3$ solution and brine, dried ($MgSO_4$) and evaporated to dryness. The crude reaction product was purified by chromatography on silica gel (50 g) (toluene/acetone, 95/5 → 8/2) to give compound 12 a (600 mg, 68%). Rf 0.60 (toluene/acetone, 8/2).

"Trisaccharide" 12b; intermediate compound according to formula VII A.

To a solution of compound 12a (460 mg, 0.34 mmol) in dry pyridine (2.0 ml) was added 4 ml of pyridine/acetic acid/hydrazine hydrate (6/4/0.5, v/v). The mixture was stirred for 6 min at room temperature. Dichloromethane (50 ml) was added and the mixture was washed with water, $NaHCO_3$ solution and brine. The organic solution was evaporated to dryness and the residue was purified by chromatography on silica

gel (dichloromethane/acetone, 97/3 → 9/1) to give compound 12b (370 mg, 87%). Rf 0.42 (dichloromethane/acetone, 9/1); $[\alpha]_D^{20}$ = +187/4° (c = 1, $CH_2CL_2$)
$^1$H NMR ($CDCl_3$):
unit 2: 4.32 (d, 1H, $J_{1,2}$ = 7,5 Hz, H-1); 3.79 (s, 3H, $COOCH_3$)
unit 3: 4.97 (d, 1H, $J_{1,2}$ = 3.6 Hz, H-1); 5.39 (dd, 1H, $J_{2,3}$ = 10.5 Hz, $J_{3,4}$ - 9.0 Hz, H-3)
unit 4: 3.72 (s, 3H, $COOCH_3$)
unit 5: 3.39 (s, 3H α-$OCH_3$); 4.72 (d, 1H, $J_{1,2}$ = 3.0 Hz, H-1); 5,22 (dd, 1H, $J_{2,3}$ = 10.5 Hz, $J_{3,4}$ = 9.0 Hz, H-3)

"Tetrasaccharide" 14b

A mixture of aglycon 12b (180 mg, 0.14 mmol), silver triflate (93 mg, 0.36 mmol), activated molecular sieves (4Å, 600 mg) and 2,6-di-tert-butylpyridine (55 µl, 0.30 mmol) in dichloromethane (5 ml) was stirred at -50 °C under nitrogen. Glycopyranosyl bromide 13b [C.A.A van Boeckel et al, J. Carb. Chem., 4, 293(1985)] (140 mg, 0.29 mmol), dissolved in dichloromethane (3 ml), was added dropwise over 15 min. The reaction mixture was stirred for 1 h at -50 °C, then diluted with dichloromethane and filtered over Hyflo. The organic solution was washed with $NaHCO_3$ solution and brine, dried ($MgSO_4$) and evaporated to dryness. The crude reaction product was eluted from a column of silica gel (dichloromethane/acetone, 98/2 → 9/1, v/v) to give impure tetramer. The isolated product was dissolved in a mixture of pyridine (6 ml) and acetic anhydride (2 ml), containing a catalytic amount of 4-(dimethylamino)-pyridine. After 1 h at room temperature, TLC analysis showed the acetylation of remaining hydrolysed bromide 13b to be complete. After evaporation of the solvents and coevaporation with toluene (3 x 10 ml), the crude mixture was chromatographed on silica gel with the same eluent. Evaporation of the appropriate fraction afforded pure compound 14b (130 mg, 49%)
$^1$H, NMR ($CDCl_3$)
unit 1: 5.50 (d, 1H, $J_{1,2}$ = 3.8 Hz, H-1); 3.26 (dd, 1H, $J_{2,3}$ = 10.0 Hz, H-2)
unit 2: 4.32 (d, 1H, $J_{1,2}$ = 8.0 Hz, H-1); 3.75 (s, 3H, $COOCH_3$)
unit 3: 5.37 (dd, 1H, $J_{3,4}$ = 9.0 Hz, H-3); 3.15 (dd, 1H, $J_{2,3}$ = 11.0 Hz, H-2)
unit 4: 3.71 (s, 3H, $COOCH_3$)
unit 5: 4.72 (d, 1H, $J_{1,2}$, 3.8 Hz, H-1); 3.39 (s, 3H, α-$OCH_3$)

"Tetrasaccharide" 15b (product of formula III)

A solution of compound 14b (80 mg, 0.050 mmol) in chloroform (3.5 ml) was added dropwise to a mixture of chloroform (2.5 ml), methanol (15 ml) and aqueous sodium hydroxide solution (3.0 ml, 4N) during 30 min. The reaction mixture was stirred for 16 h and acidified at 0 °C to pH 1.5 with aqueous hydrochloric acid (6N). The reaction mixture was extracted with dichloromethane (5 x 15 ml) and the combined organic layers were washed with ice-water (15 ml), dried ($MgSO_4$) and evaporated to dryness. The residue was chromatographed on silica gel (3 g) with dichloromethane/methanol/acetic acid (80/20/1, v/v) as eluent to give pure compound 15b (52 mg, 76%). Rf 0.70 (dichloromethane/methanol/acetic acid; 80/20/1)

"Tetrasaccharide" 16b (sulphation of free hydroxyl-groups)

Sulphur trioxide-trimethylamine complex (132 mg, 0.95 mmol) was added to a solution of compound 15b (52 mg, 0.038 mmol) in N,N-dimethylformamide (2.5 ml), and the mixture was stirred for 20 h at 50 °C under nitrogen atmosphere. The reaction mixture was applied to a column of Sephadex LH-20, equilibrated with N,N-dimethylformamide, and eluted with the same solvent. The product, which was contaminated with lower sulphated products, was dissolved in N,N-dimethylformamide (2.5 ml) and stirred again in the presence of sulphur trioxide-trimethylamine complex (150 mg, 1.08 mmol) for 20 h at 50 °C. The mixture was cooled and $NaHCO_3$ (300 mg, 3.57 mmol) in water (4 ml) was added, where upon the mixture was stirred for a further 20 min at room temperature. After evaporation to dryness, the residue was centrifugated in a mixture of dichloromethane/methanol 1.1 v/v (8 ml) and the organic layer was decanted from the salt crystals. After evaporation to dryness the residue was eluted from a column of silica gel (dichloromethane/methanol, 9/1 → 8/2) to give pure product 16b (42 mg, 58%). Rf 0.60 (ethyl acetate/pyridine/acetic acid/water 11/7/1.6/4).

"Tetrasaccharide" 17b (hydrogenolysis)

A solution of compound 16b (42 mg, 0.022 mmol) in a mixture of water (6 ml) and tert-butanol(2 ml) was stirred under a hydrogen atmosphere in the presence of 10% Pd/C (30 mg) for 20 h. The suspension was filtered and the filtrate evaporated in vacuo. The residue was dissolved in water (7 ml) and again hydrogenated for another 20 h in the present of fresh catalyst (30 mg). The suspension was filtered and concentrated to dryness to give compound 17b (27 mg, 90%).

"Tetrasaccharide" 18b [Compound of formula I]

Compound 17b (27 mg, 0.020 mmol) was dissolved in water and stirred in the presence of pyridine-sulphur-trioxide complex (10 mg) and $Na_2CO_3$ (10 mg). A second, third and fourth portion of pyridine-sulphur trioxide complex and $Na_2CO_3$ were added after 2, 4 and 6 h stirring, respectively. After stirring for 20 h the sulphation was complete. The reaction mixture was eluted from a column of Dowex WX-4 ($Na^+$) with water, evaporated to dryness and desalted on a column of Sephadex G-10 to give the desired tetrasaccharide fractions. The fractions were freeze-dried to give amorphous , white, powdery 18b (18.2 mg, 55%); $[\alpha]_D^{20}$ = +92.7° (c = 0.91, water) $^1H$ NMR data (360 MHz, $D_2O$, ref. HOD):
unit 5: 5.03 (d, 1H, H-1, J = 3.6 Hz), 3.48 (dd, 1H, H-2, J = 3.6 Hz, J = 9.4 Hz), 4.45 (dd, 1H, H-3, J = 9.4 Hz, J = 9.9 Hz);
unit 4: 4.91, 5.11 (d,d, 2H, $-OCH_2O-$, J = 7.5 Hz), 3.85, 4.12 (m, 2H, $OCH_2C$), 4.41 (t, 1H, $CHCOO^-$, J = 2.4 Hz);
unit 3: 5.12 (d, 1H, H-1, J = 3.6 Hz), 3.42 (dd, 1H, H-2, J = 3.6 Hz, J = 9.4 Hz), 4.37 (dd, 1H, H-3, J = 9.4 Hz, J = 9.9 Hz);
unit 2: 4.61 (d, 1H, H-1, J = 7.9 Hz);
unit 1: 5.61 (d, 1H, H-1, J = 3.6 Hz), 3.25 (dd, 1H, H-2, J = 3.6 Hz, J = 9.4 Hz), 3.62 (t, 1H, H-3, J = 9.9 Hz), 3.56 (t, 1H, H4, J = 9.9 Hz), 3.42 (s, 3H, $OCH_3$).

Example 2

(reference to flow sheets A and B)

Compound 14a

Compound 14a was prepared according to the method described for the preparation of 14b whereby known 3,4,6-tri-O-acetyl-2-azido-2-deoxy glucopyranosyl bromide (13a) was used instead of 13b. The temperature of the reaction was 0 °C and the reaction time 6 hours.

Compound 18a (according to formula I) chemical name:

Methyl 4-[0-2-deoxy-3,4,6-tri-O-sulfo-2-(sulfoamino)-α-D-glucopyranosyl-(1→4)-0-β-D-glucopyranuronosyl-(1→4)-0-2-deoxy-3,6-di-0-sulfo-2-(sulfoamino)-α-D-glucopyrano-syloxy-(1R)carboxylethoxy)methyl]-2-deoxy-3,6-di-0-sulfo-2-(sulfoamino)-α-D-glucopyranoside decakis sodium salt.

Compound 18a was prepared starting from 14a via intermediates 15a, 16a and 17a using the methods described for the preparation of 15b, 16b and 17b respectively. $[\alpha]_D^{20}$ = +94.5° (c = 0.2, water).

Example 3

(reference is made to flow sheet C)

Compounds 22 (a and b) (formula VIII)

Monosaccharide building block 20d, prepared by standard methods described in carbohydrate literature, is selectively α-coupled with the primary hydroxyl group of glyceric acid method ester following standard literature methods. See e.g.
H. Paulsen and W. Stenzel Chem. Ber. III, 2334(1978).
H. Paulsen, Angew. Chemie 94, 184(1982).
P. Fügedi, P.J. Garegg, H. Lönn and T. Nordberg Glyconjugate J. 4.97(1987).
R.R. Schmidt, Angew. Chemie 98, 213(1986).

The resulting compound 21 is a mixture of the diastereo-isomers (R) and (S), which can easily be separated by column chromatography yielding 21a (24%) and 21b (21%).

Both compounds 21 were methoxy methylated in an analogous manner as described for compound 4 in example 1 and then acetolysed and treated with HF/pyridine in accordance with the procedure described for compound 8 in example 1. The resulting compounds 22a and 22b were obtained in 35% yield.

Compounds 24b (formula IX)

Compounds 22a and 23 were coupled in the presence of $BF_3OEt_2$ in an analogous manner as described for compound 10a. (Ex. 1).

The resulting compound 24a (yield 71%) was treated with hydrazine hydrate in an analogous manner as described for 12b yielding compound 24b in 90% yield.

Compound 25b (formula III)

The above obtained compound 24b was subsequently coupled with compound 22b (S-form) according to the method described for 10a (Ex. 1) yielding the protected compound 25a in 44% yield. The latter compound is hydrolysed to obtain 25b.

Compound 26 (formula I)

Compound 25b is converted in compound 26 in a 3-step procedure as described in example 1 and 2. Yield 56%.

Compound 27 (formula I)

In a similar manner as described above compound 24b was coupled with 22a and further converted to compound 27 (which compound differs from 26 in the configuration of the second unit being (R) rather than (S).

**Claims**

1. A saccharide of the formula:

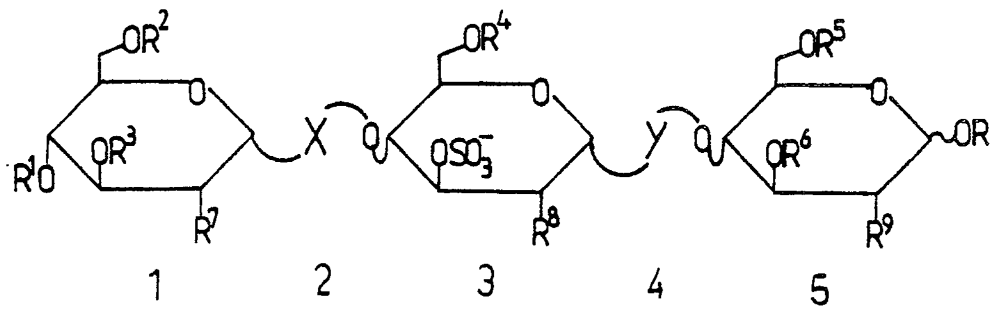

I

wherein
X and Y represent a moiety selected from an optionally sulphated uronic acid moiety and a non-uronic acid spacer which consists of a linear or cyclic chain of atoms and carries at least one electronegatively charged substituent preferably a carboxylate group, provided that at least one of the moieties X or Y represents the said non-uronic acid spacer,
R is hydrogen or alkyl (1-20 C),
$R^1$, $R^2$, $R^3$, $R^4$, $R^5$ and $R^6$ represent hydrogen or the moiety $SO_3^-$
$R^7$, $R^8$, $R^9$ represent OH, $OSO_3^-$, $NHSO_3^-$ or NH-acyl and the charge of the various charged moieties is compensated by suitable pharmaceutically acceptable counter-ions.

2. A saccharide according to claim 1 in which - whether or not in combination - :
Y or both X and Y represent(s) the spacer moiety of formula II:

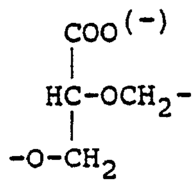

R represents an alkyl moiety with 1-4 carbon atoms and preferably methyl,
$R^2$ and $R^5$ represent the $SO_3^-$ moiety;
$R^7$, $R^8$ and $R^9$ and $NHSO_3^-$ or $OSO_3^-$.

3. A saccharide according to claim 1 of the formula:

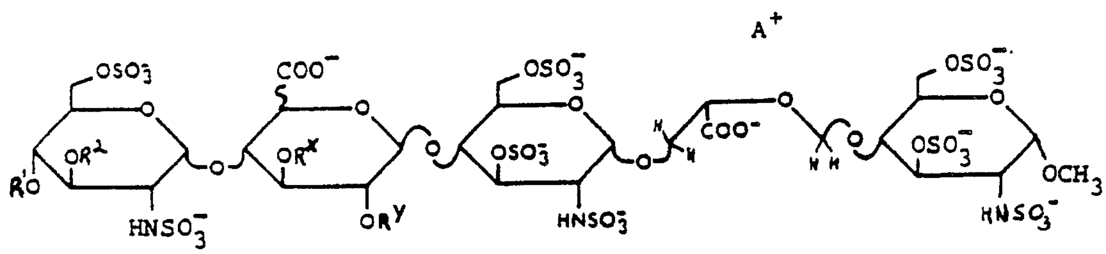

whereby A is a suitable kation,
$R^1$ and $R^2$ have the meanings indicated in claim 1, and
$R^x$ and $Y^y$ represent hydrogen or the moiety $SO_3^-$.

4. Saccharide according to claim 3 wherein the kation is an alkali metal kation, preferably the sodium-ion.

5. Process for the preparation of a saccharide according to claim 1 characterised in that the said saccharide is prepared by methods in actual use or described in the literature.

6. A saccharide intermediate compound according to formula III or a salt thereof.

7. A saccharide intermediate of formula V or a salt thereof.

8. A saccharide intermediate of formula VII A, VII B or VII C or a salt thereof.

9. A saccharide intermediate of formula VIII or a salt thereof.

10 A saccharide intermediate of formula IX or a salt thereof.

11 A pharmaceutical preparation containing the saccharide of claim 1 in admixture with one or more suitable carriers or diluents.

Flow sheet A

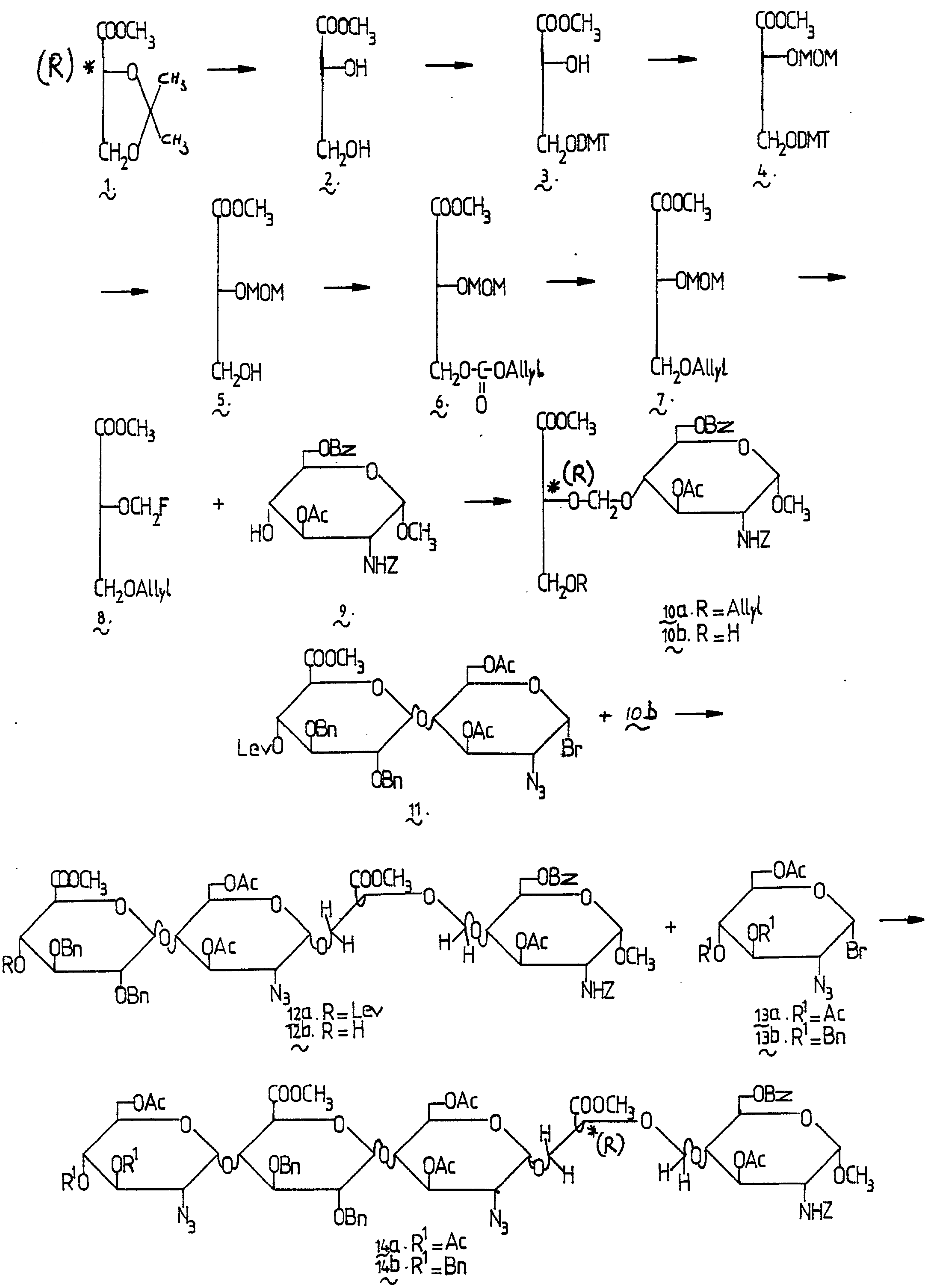

1. 2. 3. 4. 5. 6. 7. 8. 9.

10a. R = Allyl
10b. R = H

11.

12a. R = Lev
12b. R = H

13a. $R^1$ = Ac
13b. $R^1$ = Bn

14a. $R^1$ = Ac
14b. $R^1$ = Bn

Flow sheet B

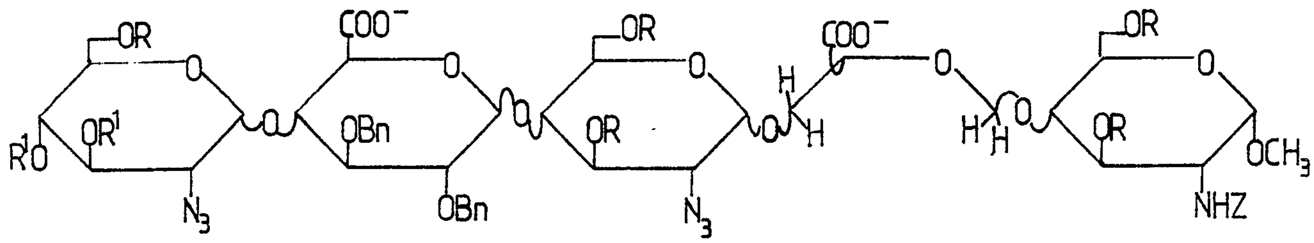

15a. $R, R^1 = H$
15b. $R = H, R^1 = Bn$
16a. $R = SO_3^-, R^1 = SO_3^-$
16b. $R = SO_3^-, R^1 = Bn$

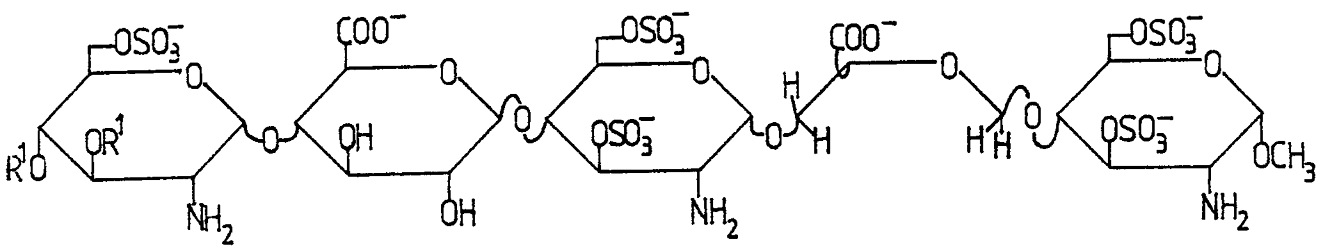

17a. $R^1 = SO_3^-$
17b. $R^1 = H$

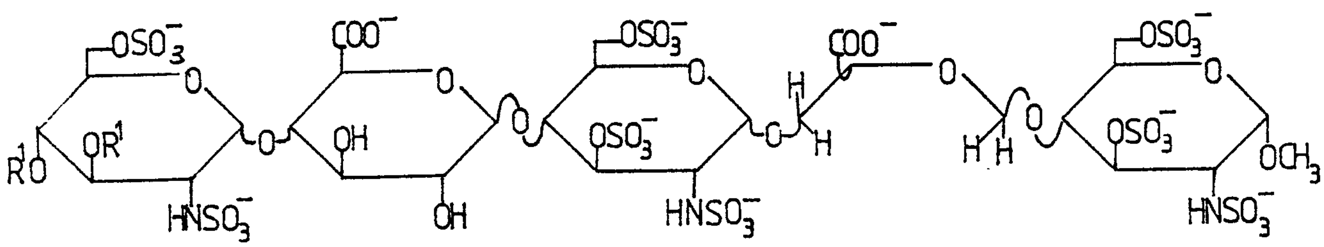

18a. $R^1 = SO_3^-$
18b. $R^1 = H$

## Flow sheet C

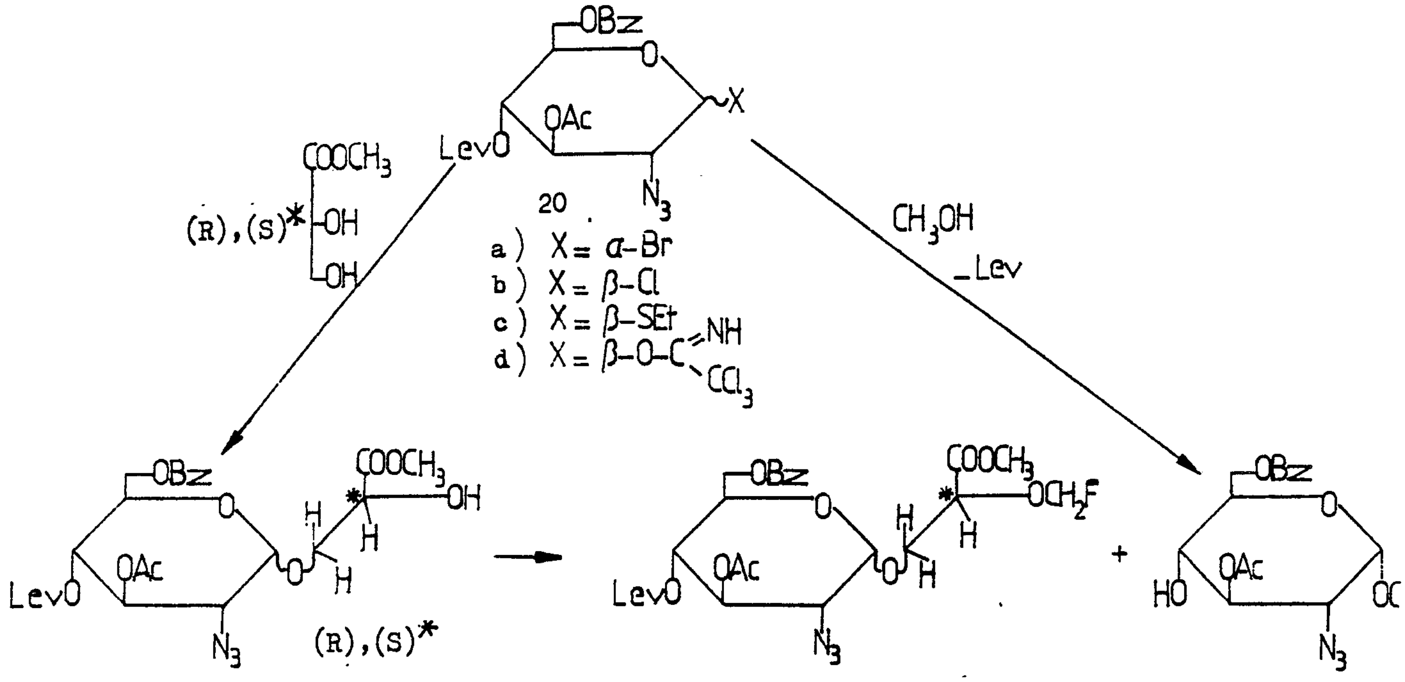


20

a) X = α-Br
b) X = β-Cl
c) X = β-SEt
d) X = β-O-C(=NH)CCl3

(R),(S)*

21 a = (R)*
b = (S)*

22 a = (R)*
b = (S)*

23

22a + 23

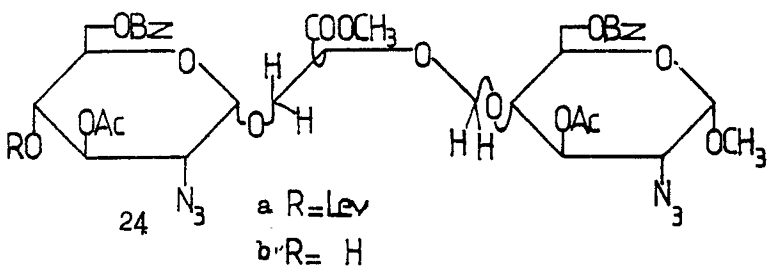

24

a R=Lev
b R= H

24 b + 22.b

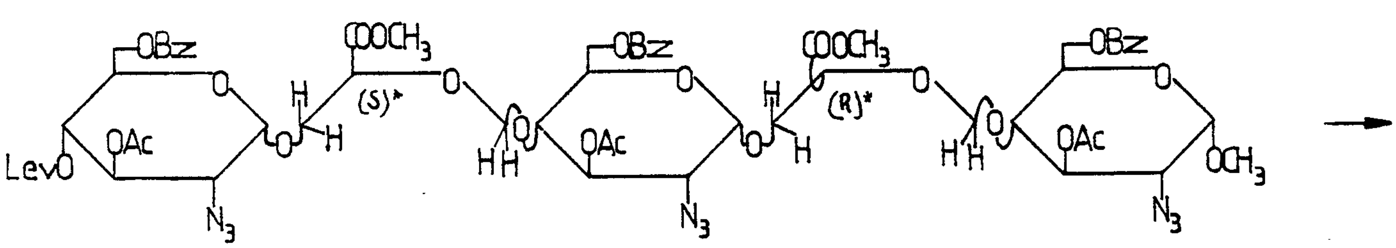

25 a

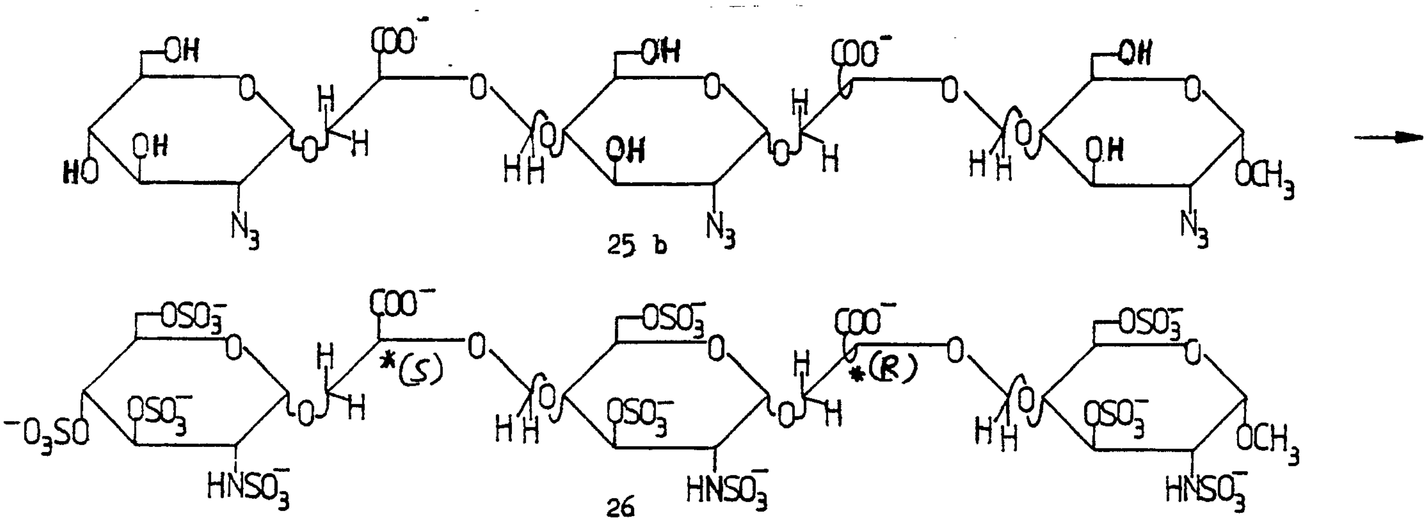

25 b

26

27 = unit ② in (R) configuration

<u>Abbreviations used in flow sheets A, B, and C.</u>

| | |
|---|---|
| (S), (R) | configuration as defined by Cahn Ingold Prelog Convention |
| DMT | 4,4'-dimethoxy trityl |
| MOM | methyloxy-methyl |
| BZ | benzoyl |
| Ac | acetyl |
| Z | benzyloxycarbonyl |
| Lev | levulinoyl |
| Bn | benzyl |

European Patent Office

# EUROPEAN SEARCH REPORT

Application Number

EP 89 20 1441

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | EP-A-0 084 999 (CHOAY) * Page 1, line 1 - page 6, line 12; claims 26-33 * | 1,11 | C 07 H 15/04<br>A 61 K 31/70 |
| P,X | ANGEWANDTE CHEMIE (International Edition in English), vol. 27, no. 9, 1988, pages 1177-1178, VCH Verlagsgesellschaft mbH, Weinheim, DE; C.A.A. VAN BOECKEL et al.: "A synthetic heparin-like compound which still activates antithrombin III although it contains an open chain fragment instead of alpha-L-idopyranuronate" * Whole article * | 1-11 | |
| | | | **TECHNICAL FIELDS SEARCHED (Int. Cl.4)** |
| | | | C 07 H 15/00<br>C 07 H 11/00<br>A 61 K 31/00 |

The present search report has been drawn up for all claims

| Place of search | Date of completion of the search | Examiner |
|---|---|---|
| THE HAGUE | 08-08-1989 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)